# EUROPEAN PATENT SPECIFICATION

(11) **EP 0 839 779 B1**
(45) Date of publication and mention of the grant of the patent: **25.07.2001**
(21) Application number: 97850144.3
(22) Date of filing: 21.10.1997
(51) Int. Cl.: C04B 41/00, C04B 41/53, E04G 23/02, G01N 33/38

(54) **A process for surface treatment and measuring method usable therein**
Verfahren zur Oberflächenbehandlung und bei dem Verfahren verwendbare Messmethode
Procédé de traitement de surface et méthode de mesurage utilisable dans ledit procédé

(30) Priority: 24.10.1996 SE 9603896
(43) Date of publication of application: 06.05.1998
(73) Proprietor: Ekologisk Teknik Mats Holmström AB, 117 36 Stockholm (SE)
(72) Inventor: Holmström, Mats, 162 20 Vällingby (SE); Holmström, Ingmar, 117 40 Stockholm (SE); Pühringer, Josef, 183 62 Täby (SE)
(74) Representative: Larsson, Kjell

(56) References cited:
- GB-A- 2 276 159

## Description

### Introduction

The present invention relates to a process for protecting the surface of a material member, for example a structural member, from the damaging action of water-soluble salts present therein. The invention also relates to a process for cleaning a surface as stated above from salts which may have a damaging effect on the surface. In addition, the invention relates to a method for determining the surface moisture content of a material, which method is particularly usable in the processes according to the invention.

### Background and Presentation of Problems

One often sees that interior surfaces and facades of older buildings disintegrate over time, for example by the surface becoming discoloured and damaged, layers of plaster becoming detached, stucco deteriorating, etc. It has been known for a long time that such damage can be caused by water-soluble salts which have penetrated into the structural materials. The salts can come from, for instance, saline water, rain water, the groundwater in the soil, or activities which are taking place or have taken place on the premises. It is also known that such damage occurs as a result of the salts changing form owing to external factors. Since these water-soluble salts accumulate mainly in the surface layers of the materials, this is where the greatest damage occurs. The effect of the presence of the salts is that some characteristics of the materials change. Accordingly, the result may be the decay of the material surface, but the materials may also become more brittle, their appearance may change, etc. Materials which are damaged by the action of salt are e.g. mineral materials, such as masonry, mortar and plaster, natural stone, and concrete. The problems become particularly serious when the building exposed is of historical interest since in these cases it is especially important to preserve the original materials and appearance.

In order to solve the problems defined above, one has mainly employed measures which involve the temporary restoration of a surface, after which it is again exposed to the same damaging action until it needs to be restored again. Naturally, this is not a practical solution if the purpose is permanently to have surfaces which are as well preserved as possible, for example in the case of interior surfaces, building facades, and monuments which are continually on view. Moreover, considering the above-mentioned deterioration of the properties of the materials, a permanent solution is desirable.

Consequently, there have been attempts at finding permanent solutions which not only restore the surface but also prevent new damage from occurring. However, all the processes used so far have resulted in some type of secondary damage.

A known process for reducing the damage caused by water-soluble salts is the application of so-called sacrificial renders. The purpose of a sacrificial render is to extract the salts from the wall surface to the sacrificial render, so that the damage occurs there instead of in the original material surface. The sacrificial renders can consist of e.g. porous hydrophobic materials, so-called cleaning plasters. A drawback of this process is that the renders have to be replaced at regular intervals, which means that the material is put under great stress. In addition, the damage caused by the salts may still extend into the original material since the salt-active level depends on the uncontrolled climate in the ambient air. Moreover, in general, the adhesion of the sacrificial renders stresses weak materials mechanically.

Another known process is the application of saturated packings of materials such as fine clays, paper, textiles, cellulose fibre and the like through which the salt is extracted. However, this process has not worked satisfactorily in instances where the material surface comprises several different salts, something which is very often the case. Moreover, it is an additional drawback that the high moisture load which is achieved in this process can cause further damage to a material which is already laden with salt. As in the case of the sacrificial renders, the extraction of the salts cannot with certainty be guided to the packing and the underlying salt-laden material may also be damaged. The extraction effect of the process is generally poor.

Another difficulty which one has also come across when using the methods described above concerns the fact that in order to be able to apply a suitable material as stated above, one has to know which types of salt the surface comprises. This requires an analysis of the surface, which analysis with present technology has to be performed in a laboratory. Consequently, one first has to take a sample of the surface layer which is to be restored and then transport it to a suitable location. This is damaging to the surface as well as time-consuming and costly.

Other presently known processes utilise a special effect for forming salt growth on surfaces of porous materials, more particularly, a type of surface diffusion on the surfaces of the salt formations.

It is known from EP 0 111 767 that it is possible to reduce the damaging effect of water-soluble salts by adding substances dissolved in water to the surface of the material, which change the saturation vapour content and surface tension of noxious saline solutions. However, this process, too, presupposes that it is known which types of salt are present in the material, which, as mentioned above, normally requires a thorough analysis in a laboratory.

Another drawback associated with the processes mentioned above is that they change the function and appearance of the surface of the material in a non-reversible manner, which, for example, is not acceptable in modern conservation whose purpose it is to document a historical development. In addition, the known processes weaken material surfaces which are already strained.

Thus, so far, it has not been possible to protect and/or restore surfaces in such a way that the effect is acceptable to those working in conservation. Nor has it been economical to treat other large surfaces, such as room surfaces and building facades since, with the known processes, it has been impossible to prevent further damage.

With respect to the part of the invention which concerns determination of the saturation vapour content of the material, the method most commonly used today is, first, for a sample to be taken of the material in question and, then, for a laboratory analysis of this sample to be performed in order to determine which salts have been deposited in the material. When this analysis has been performed, and consequently it has been established which salt or salts are deposited and in what amounts, a calculation of the total saturation vapour content for the salt or mixture of salts is done with the aid of standard tables of the saturation vapour contents of salts. This method exhibits three main drawbacks, namely: 1) it is relatively costly and time-consuming to send a sample of material away for analysis in a laboratory; 2) a relatively poor approximation of the actual saturation vapour content is obtained since the saturation vapour content of a mixture of salts does not depend linearly on the salts included therein and, consequently, it is difficult to calculate the total saturation vapour content for such a mixture of salts, and 3) the taking of samples contributes to the degradation of the material since it is necessary to remove the sample from the same.

### General Description of the Invention

The present invention solves the problems described above by providing a process for protecting the surface of a material member, preferably a structural member, from the damaging action of water-soluble salts present therein. The process comprises the following steps: assessing the moisture content of the salt or salts present in the surface layer of the material; providing a buffer zone between the surface layer and the environment; and, finally, regulating the climate in the buffer zone so that the salts assume a state such that their damaging action is essentially eliminated.

The invention also relates to a process for cleaning a material surface of salts present therein, which salts, as in the cases mentioned above, may have a damaging effect on the surface.

Finally, the invention also relates to a method for determining saturation vapour content, which method is particularly usable in both processes mentioned above and which solves or at least essentially eliminates the problems described above in connection with prior art for determining saturation vapour content.

### Detailed Description of the Invention

More particularly, the invention relates to a process for protecting the surface of a material member, preferably a structural member, from the damaging action of water-soluble salts present therein, characterised by the following steps
assessing the saturation vapour content of a salt or salts present in the surface layer of the material,
providing a buffer zone between the surface layer and the environment, the climate in the buffer zone being isolated from the environment, and
on the basis of the assessed saturation vapour content, regulating the climate in the buffer zone so that the moisture content of the surface layer reaches a value where the salt or salts assume a state such that their damaging action is essentially eliminated.

The establishment of this state, which can be called the ideal state, in a material is a revolutionary discovery in the field of surface treatment and elimination of salt damage. Previously, it has been known that the restructuring of the salts has a damaging effect on e.g. a wall material. Now, according to the invention, it has been discovered that this restructuring as well as changes in the state of aggregation of the salts can be eliminated and/or regulated by the arrangement of a suitable moisture gradient in the surface layer. In other words, these changes in the salts in materials occur because they are constantly striving to achieve the ideal state. The ideal state can be achieved in solution form or in solid form, depending on the ambient conditions. What is essential according to the present invention is the stabilisation of the climatic conditions so that the salts maintain an ideal state and consequently do not change their state of aggregation.

The invention also relates to an embodiment of the process described above, wherein the saturation vapour content of said surface layer is assessed by the supplying of moisture to at least a part of it until it is in a saturated state, after which the saturation vapour content is measured in situ. For example, moisture has to be supplied when the surface layer has previously been exposed to drying conditions. However, by choosing a suitable measuring method it is possible to limit the wetting to a very small area. If the preferred measuring method according to the invention described below is used, it is for example sufficient to wet a very small area with a few drops of water. On the other hand, in some cases it may be advantageous to dry at least part of the surface layer until it is essentially dry prior to assessing said saturation vapour content and then to measure the saturation vapour content directly on the material member.

According to a preferred embodiment of the process according to the invention, a buffer zone is achieved by the arrangement of a diffusion-retarding or vapour-tight barrier between the buffer zone and the environment. Depending on the ambient climate, the barrier may be combined with a temperature gradient. The temperature gradient can be achieved by the buffer zone or its boundary layer having heat-insulating properties. The thickness of the buffer zone can be adjusted to the conditions existing at the site, e.g. from a few millimetres to a few decimetres, preferably from about 0.5 cm to about 3 cm. The barrier can be made of e.g. plastic, metal, or a mineral material. In order for the regulation of the climate in the buffer zone to function satisfactorily, the barrier should also be adapted to the choice of regulating method. Since the process is primarily intended as a permanent solution, and, consequently, the barrier is intended to be permanently arranged adjacent to the material surface, it may be suitable, on the side of the barrier which faces away from the material layer, i.e. into the room or away from the facade, to apply a suitable layer of plaster, or the like, in order to make the appearance of the material surface with the applied barrier resemble the original appearance as much as possible. In particular, this may be done when building facades and walls are concerned. Thus, the difference between the layers according to the invention and the sacrificial renders made of e.g. plaster discussed above, is that, by virtue of the buffer zone, the layers which according to the invention are applied to the barrier are not at all affected by the state of the salts in the material surface.

According to an embodiment of the process according to the invention, a heat-insulating spacing material is arranged in the buffer zone, which material preferably has open porosity. In addition, the material should preferably be hydrophobic. Optionally, the porosity of the material may vary in the buffer zone. The open porosity allows the spacing material to absorb salts which may migrate from the surface layer, since it has been observed that even when the salts are in the ideal state a very slow migration of the same occurs through the surface layer. However, this occurs at an extremely slow pace in the conditions according to the invention and can therefore otherwise essentially be disregarded. Preferably, according to the invention, the spacing material is applied in the form of sheets or mats, which may comprise fibres or particles.

According to an embodiment which is particularly advantageous for certain applications, the buffer zone comprises air only. In this case, the buffer zone is arranged with e.g. stays and a barrier consisting of a rigid sheet.

According to a special embodiment of this process according to the invention, glass, mineral wool, plastic or metal is used as a spacing material. However, the spacing material may be selected among other materials as long as the desired objectives are achieved, that is, a support for the barrier and a recipient of salts. In addition, the spacing material prevents salts from reaching the barrier. The fixing to the salt-containing surface layer can be achieved by stays, with or without pressure distribution. The distribution of pressure can also be achieved by nets of a suitable dimension, wire diameter, and mesh size, preferred materials being stainless or rust-resistant steel, plastic, glass fibre or metal which has been treated with plastic and protected against corrosion, or a combination of these materials. The layer of spacing material can also be anchored to the salt-containing surface by means of pins or bolts. As mentioned above, a further purpose of the spacing material is heat-insulation, so as to improve the possibilities of keeping the climate constant in the buffer zone. It may also be possible to apply supplementary insulation to the side of the barrier which is opposite the spacing material in order further to improve the climate regulation of the buffer zone.

According to a preferred embodiment of the process according to the invention, the climate in the buffer zone is regulated by the adjustment of the moisture content therein. Depending on which state the material is in, the moisture content is adjusted by being increased or decreased, so as to reach the value at which the salts assume the ideal state.

According to a special embodiment of the process according to the invention, the moisture content of the surface layer of the material is adjusted to a value which essentially corresponds to the saturation vapour content of the salt or salts present therein. The person skilled in the field will appreciate that this value is preferably somewhat higher than the saturation vapour content rather than somewhat lower, but that a stable value is crucial in order to avoid detrimental changes in the state of aggregation of the salts. Stable values which are somewhat below the saturation vapour content will also allow the salts to assume states which are close to the ideal states. The adjustment is performed on the basis of the assessment of the saturation vapour content which was carried out according to the first step in the process according to the invention. The skilled person will also appreciate that this adjustment can mean either increasing or decreasing the moisture content, depending on the original state of the surface layer.

According to an alternative embodiment of the process according to the invention, the climate in the buffer zone is regulated by the adjustment of the temperature therein, which more particularly is achieved by a temperature gradient being provided therein.

According to another alternative embodiment of the process according to the invention, the climate in the buffer zone is regulated by the adjustment of the vapour pressure therein.

According to yet another alternative embodiment of the process according to the invention, the climate in the buffer zone is regulated by the supplying of conditioned air.

It is also feasible to achieve suitable values with respect to vapour pressure, temperature, or moisture content by means of control equipment. In such a case, the condition of the surface layer of the salt-containing material is determined by measuring the surface temperature or surface moisture content, and then the desired climate is achieved, e.g. by supplying air which is suitably conditioned. Measuring the moisture content and temperature of the surface layer can also be performed by means of conventional insert measuring devices.

The present invention also relates to a process for cleaning a material surface, such as a brick wall without plaster or a wall which has or has not been painted, of water-soluble salts present therein, the process being characterised by the following steps
assessing the saturation vapour content of the salt or salts present in the surface layer of the material,
providing a buffer zone between the surface layer and the environment, the climate in the buffer zone being insulated from the environment, and
on the basis of the assessed saturation vapour content, regulating the climate in the buffer zone so that the moisture content of the surface layer reaches a value where the salt or salts assume a state such that they are extracted by migrating through the material towards the surface of the same. The physical background to this extraction is a moisture gradient which occurs in the material, wherein the salts first absorb moisture and then as a result of evaporation, and the structural change this causes, migrate to the material surface.

Consequently, this special process is a process which is intended to be carried out on one occasion, the climate set in the buffer zone being maintained during a period of time which is sufficiently long to allow a considerable part of the harmful salts which may be present in the surface to be extracted.

According to a preferred embodiment of this second process according to the invention, said saturation vapour content in the surface layer is assessed by moisture being supplied to at least part of it until it is in a saturated state, after which the saturation vapour content is measured in situ. As mentioned above in connection with the first process according to the invention, moisture has to be supplied only in cases where the moisture content of the surface layer is low because of prior conditions. By choosing a suitable measuring method one can limit this wetting to a small area in this case as well. For example, if one uses the preferred measuring method according to the invention described below, it is sufficient to wet a very small area with a few drops of water. On the other hand, in some cases it may be advantageous to dry at least part of the surface layer until it is essentially dry prior to assessing said saturation vapour content and then measure the saturation vapour content directly on the material member.

According to an embodiment of the process as stated above, the salts are extracted from the salt-containing material through a sacrificial render of a mechanically weak material with open porosity (e.g. clay, cellulose fibre), which is applied to the material and which is easy to remove. As a result, the risk of damaging very weak surface layers is reduced.

According to an embodiment of the process as stated above, the buffer zone is achieved by the arrangement of a diffusion-inhibiting or diffusion-proof barrier between the buffer zone and the environment, which barrier is made of e.g. plastic, metal or a mineral material. In cases where the process is being carried out on a surface which is of cultural interest, it may be desirable to use a barrier material which is transparent, so that the surface can be viewed during the actual cleaning period as well. The thickness of the buffer zone varies depending on the character of the surface which is to be cleaned, what the environment around the surface is like with respect to space and what one wishes to achieve by the cleaning. However, it will be appreciated that a thicker buffer zone may be preferable in many cases, since it may then be easier to maintain a constant climate. For example, the buffer zone can be about 1 m.

According to a preferred embodiment of the above-mentioned second process according to the invention, the buffer zone contains air only.

According to another embodiment of the process, a heat-insulating spacing material is arranged in the buffer zone, which material preferably has open porosity and in addition preferably is non-hydrophilic. The purpose of the heat insulation is to maintain the set climate while the main purpose of the open porosity of the material is to collect extracted salts.

The spacing material mentioned above can, for example, be glass, mineral wool, plastic or metal, examples of mineral wool being glass wool or rock wool.

According to an alternative embodiment of this second process according to the invention, the climate in the buffer zone is regulated by adjusting the moisture content thereof.

According to a special embodiment of the process as stated above, the moisture content of the surface area of the material is adjusted to a value which is about 10% lower, preferably about 5% lower, than the saturation vapour content of the salt or salts present in the surface layer. Obviously, it is the original moisture content of the material surface which determines whether the adjustment involves an increase or a decrease in the moisture content.

According to an embodiment of the process mentioned above, said moisture content is maintained in the surface area until salts have crystallised on the surface, after which the crystals are collected from the surface. Normally, one defers the collection until the extraction has ceased, although, in some cases, it may be necessary to remove crystals several times during a cleaning if the salt content of the material is very high to begin with. Optionally, inspection openings or removable areas of the barrier and spacing material may be arranged when the buffer zone is provided in order to facilitate the inspection of the progress of the extraction.

Accordingly, when carrying out this second process according to the invention it is preferable to remove the barrier subsequent to a satisfactory cleaning. The treatment period should be long enough for the major part of the salts present in the surface to be extracted and, consequently, the process does not need to be repeated until a very long time has passed, if at all. Since it is not necessary to have a barrier permanently arranged, this process is intended especially for surfaces whose appearance is of primary interest, such as murals. It is also feasible to use the process to extract salts from old paintings or cloths, which are at risk of being destroyed.

The present invention also relates to a method for determining the surface moisture content of material surfaces, which method is of particular interest in the process according to the invention. Accordingly, the assessment of the saturation vapour content which is made in the processes according to the invention can, for example, be performed in the form of a determination according to said method or according to some other known method. However, the assessment can also be made in the form of an estimation, e.g. visually, by a person skilled in the field.

The method according to the invention, is characterised in that, in order to measure the saturation vapour content of the surface layer of a material, preferably the surface layer of a structural member, the following steps are performed:
1) inspecting said surface layer in order to determine whether there is visible surface moisture,
2a) if the surface layer is clearly wet, i.e. exhibits surplus moisture, drying a part of this surface layer,
2b) if the surface layer is clearly dry, wetting a part of this surface layer,
3) measuring the vapour content of the surface by means of a humidity meter,
   again performing 2a) or, alternatively 2b) depending on whether the surface layer is now clearly wet or clearly dry,
   repeating steps 2a) or, alternatively 2b), and 3) until a stable measured value for the surface moisture is obtained during a number of successive measurings,
4) using this stable measured value as an approximation of the saturation vapour content of the salts in the surface layer.

### Brief Description of the Drawings

Figs 1a-b illustrate how a salt-containing material surface can be protected from the damaging action of the salts present therein by means of a process according to the invention, wherein a buffer zone has been provided adjacent to the material surface by means of a barrier.

Figs 2a-c show examples of how a salt-containing material surface can be cleaned by means of a process according to the invention by the use of barriers which have been fixed to the material surface in various manners.

Figs 3a-b show examples of how a process according to the invention can be realised with various sealing sheets and various materials in the buffer zone.

Fig. 4 shows a further example of how a salt-containing material surface can be protected according to the invention, whereby a layer of plaster is applied to a barrier,

Figs 5a-c show several examples of how a surface can be protected according to the invention by the use of various spacing materials and insulating layers.

Fig. 6 shows an example of how a process according to the invention can be realised with climate regulation and a damper.

Fig. 7 shows an example of how a process according to the invention can be realised with feed-back climate regulation.

Figs 8a-b show an example of how the process according to the invention can be carried out in a cellar-vault where the walls comprise salts.

Fig. 9 schematically shows a preferred embodiment of a set-up for determining surface moisture content according to the present invention.

Figs 10a-b illustrate how moisture migrates when a humidity meter which has lower and higher vapour concentrations respectively than a material surface is applied to said material surface.

Fig. 11 is a chart showing the readings of a humidity meter depending on time from the commencement of a measurement on a material surface until a stable measured value is obtained, where the material in question has not absorbed any water-soluble salt.

Fig. 12 shows readings corresponding to those in Fig. 11, but in this case the material has absorbed a water-soluble salt or a mixture of water-soluble salts.

### Detailed Description of the Figures

In the following description of the figures the same reference numerals are used for each of the details in all the figures. Although some of the figures illustrate how salts are maintained in their ideal state in the material surfaces and other figures illustrate how salts are extracted from material surfaces, it will be appreciated that the devices employed to achieve these two objects according to the invention can to all intents and purposes be used interchangeably. Accordingly, a procedure which is described in one figure in connection with salt extraction could just as well be used in a process where the ideal state of the salts is aimed at and vice-versa. For preferred embodiments, please refer to the detailed description of the invention.

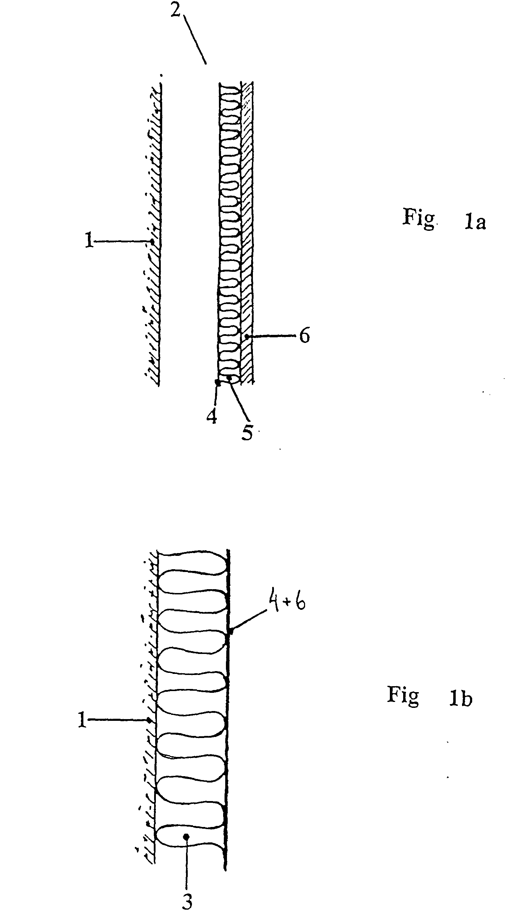

Figs 1a-b illustrate how a process according to the invention can be used to eliminate the damaging action of salts on a salt-containing material surface 1 by the provision of a buffer zone 2, wherein the climate can be regulated so that the salts in the material surface 1 assume an ideal state. More specifically, Fig. 1a shows a salt-containing material surface 1, a buffer zone 2 with a stable, regulated vapour content related to the saturation vapour content of the salts, a barrier 4, a layer 5 with heat-insulating and moisture-retarding properties and a layer 6 for appearance and mechanical strength. Fig. 1b illustrates how one can arrange a heat-insulating layer of a moisture-retarding spacing material 3 with open porosity adjacent to the salt-containing material surface 1.

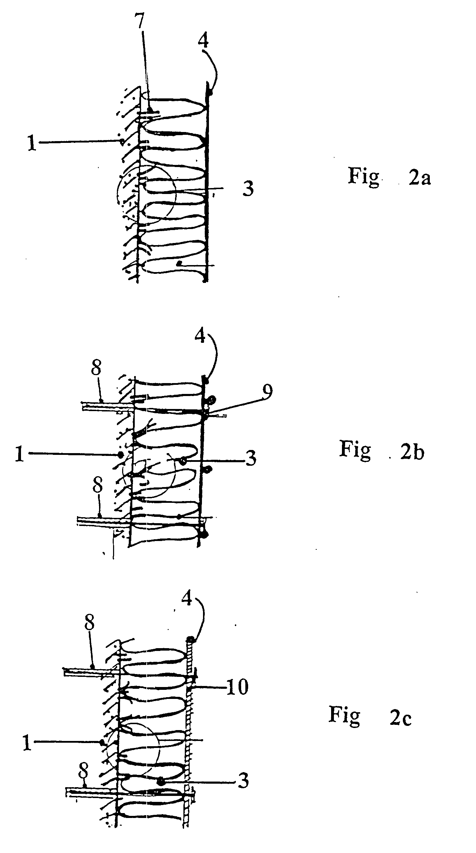

Figs 2a-c show examples of how a salt-containing material surface 1 according to the invention can be protected from salt damage by a barrier 4 and a layer 3 of heat- and moisture-regulating spacing material with open porosity being fixed thereto. Extracted salts 7 are also shown. More specifically, fig. 2b illustrates how the barrier 4 can be anchored with anchoring means 8 and a fixing net 9, while Fig. 2c shows how the anchoring means 8 can be used in conjunction with an anchoring sheet 10.

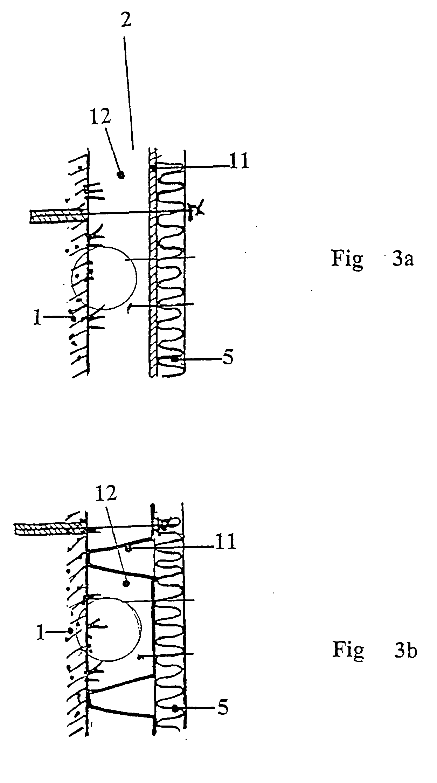

Figs 3a-b show further examples of how the process according to the invention can be realised with various sealing sheets and materials in the buffer zone 2. More specifically, Fig. 3a shows a flat sealing sheet 11 in conjunction with a buffer zone 2 comprising a heat and moisture-regulating layer of air 12, while Fig. 3b shows a profiled sealing sheet 11 with the same layer of air 12 in the buffer zone 2. In addition, both figures show an insulating layer 5 as supplementary insulation.

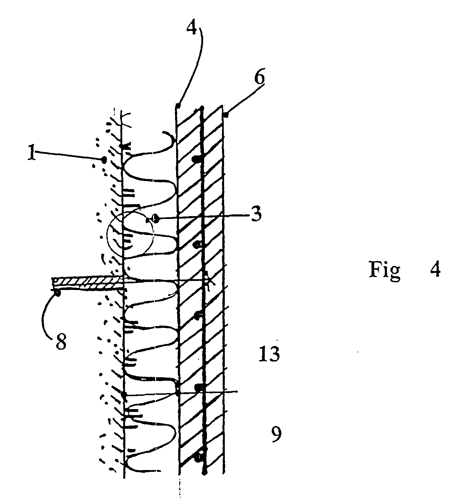

Fig. 4 shows a further example of how a salt-containing material surface 1 can be protected according to the invention. A layer of spacing material 3 has been applied to the material surface 1, which layer is kept in place by means of anchoring means 8, a reinforcement means 13 and a net 9. A layer of plaster 6 has been applied to the barrier 4 to improve its appearance.

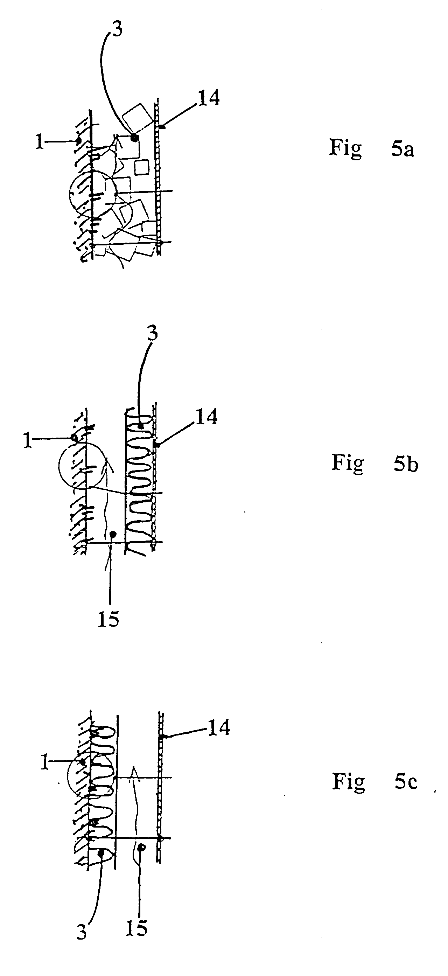

Figs 5a-c show several examples of how a surface can be protected by means of a process according to the invention by the use of various spacing materials and insulating layers. More specifically, Fig. 5 shows a material surface 1 with a layer of spacing material 3 with coarse porosity and a sealing layer 14. Figs 5b and 5c, more specifically, show how one can apply to a salt-containing material surface 1 a layer of air 15 in the buffer zone 2 in conjunction with a layer of spacing material 3, with either the layer of air 15 closest to the material surface 1 according to Fig. 5b or the spacing material 3 closest to the material surface 1 according to Fig. 5c.

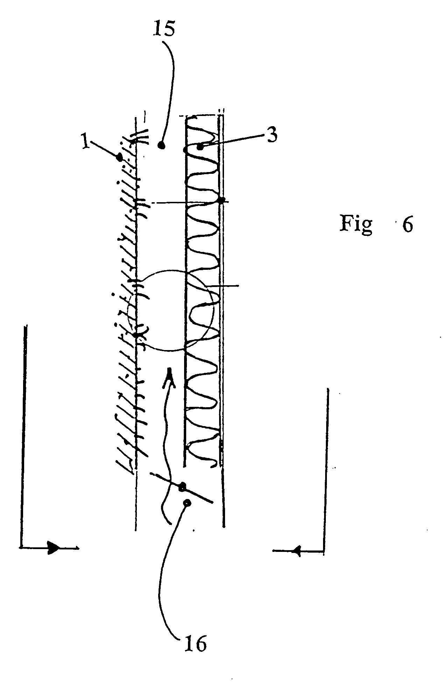

Fig. 6 shows an example of how a process according to the invention can be realised with climate regulation and a damper 16. The climate is regulated in a layer of air 15 closest to a salt-containing material surface 1, which layer is insulated by means of a layer of spacing material 3.

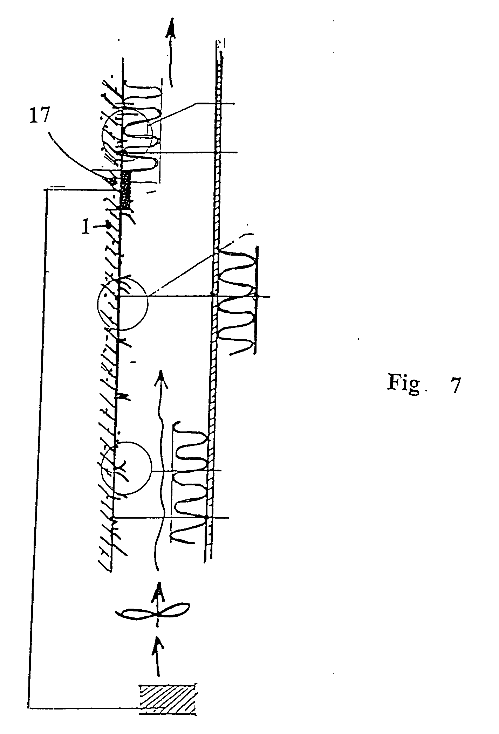

Fig. 7 shows an example of how a process according to the invention can be realised by feed-back climate regulation by means of a sensor 17.

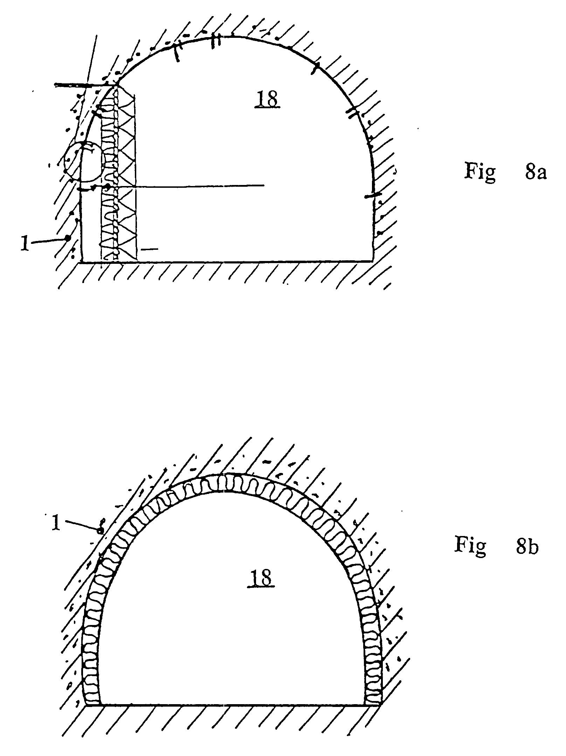

Figs 8a-b show examples of how a process according to the invention can be performed on salt-containing walls in a cellar-vault 18. More specifically, Fig. 8a shows how the process is performed on a limited wall area, while Fig. 8b illustrates how the process can be carried out on the entire wall area.

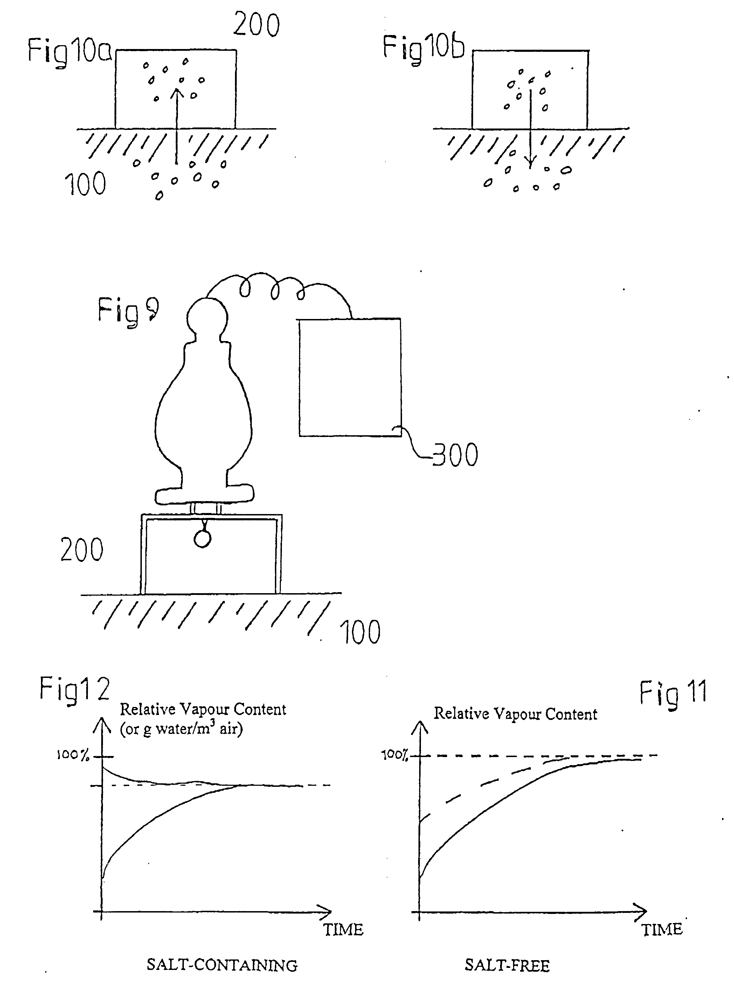

Fig. 9 shows an embodiment of a set-up for determining the saturation moisture content of a material surface 100 according to the present invention, comprising a meter cell with a transducer 200, for example a TestoTerm 452 with a hand adapter on the RF transducer, and an instrument 300 for registering and/or displaying the measured values obtained.

Figs 10a-b show how moisture migrates to or from a material surface from or to a humidity meter 200 depending on whether the wall 100 or the meter 200 has the highest vapour pressure when they are caused to contact one another.

Fig. 11 shows how the measured values obtained from the meter 200 change over time in the case of a material surface which has absorbed only negligible amounts of water-soluble salt. Two different graphs show two different initial moisture content levels of the humidity meter when it is caused to contact the material surface. As the figure shows, the measured values increase and move asymptotically towards a relative vapour content of 100%. This measured value is used as an approximation of the saturation vapour content of the material surface and indicates that the material has absorbed only negligible amounts of water-soluble salt.

Fig. 12 shows how the measured values obtained from the meter 200 change over time in the case of a material surface which has absorbed considerable amounts of water-soluble salt. Two different graphs show two different initial moisture content levels, one being higher than the saturation vapour content of the material surface and the other being lower than this saturation vapour content, of the humidity meter when it is caused to contact the material surface. As the figure shows, the measured values in both cases move asymptotically towards a measured value P that is distinct from 100%, which indicates that the material has absorbed a water-soluble salt or a mixture of water-soluble salts which have a saturation vapour content of P.

### EXAMPLES

### Example No. 1: Treatment of a wall

A brick wall has been exposed to contact with salt water for a long time and clear signs of salt damage in the surface layer of the wall can be seen.

First, the surface moisture content is determined directly on site by the use of the measuring method according to the present invention.

Then, a buffer zone is provided by the arrangement of a layer of the spacing material Enkadrän, which consists of thin plastic threads, on the wall. This layer is fixed to the wall with a non-hygroscopic glass-fibre fabric. The thickness of the buffer zone in this case is 10 mm.

One then adjusts the saturation vapour content obtained in the manner described above by adapting the permeability of the barrier with respect to the prevailing ambient climate and the temperature gradient which is formed over the buffer zone and the new surface layer.

In order to copy the original appearance of the wall, one subsequently applies a layer of plaster which is adapted to convey the same impression as the original.

### Example No. 2: Extraction of Salts

A wall surface of an older building has been damaged by the action of salts present therein. In this case, it is preferable to perform an extraction of the salts so that the wall surface subsequently can be completely restored to its original state.

Just as in the above example, first, the vapour concentration of the surface in the wall surface is measured by means of the measuring method according to the present invention.

Subsequently, one provides a buffer zone which is about 5 cm thick by attaching with stays a plastic sheeting parallel to the material surface.

The climate is regulated by adjusting the moisture content in the buffer zone, which is carried out by means of dehumidification or humidification of the air therein, depending on the prevailing climate.

### Example No. 3: Measurement of Saturation Vapour Content

A brick wall has been exposed to contact with various water-soluble salts for a long time and clear signs of salt damage in the surface layer of the wall can be seen. We want to determine the saturation vapour content of the salt or the mixture of salts which has caused the damage. First, the wall is examined visually and tactilely (i.e. one looks at and touches the wall) in order to determine if the wall seems wet, damp or dry. In this instance, wet means soaking wet and damp means that it feels damp to the touch without surplus liquid accumulating on the surface; what dry means should not require any explanation. In our case, the wall is found to be dry, and therefore we wet it locally somewhat and measure its vapour content with a conventional humidity meter. The measured value obtained is registered and we examine the wall again and it is found to be damp. In order to receive confirmation that the measured value we have obtained is a good approximation of the saturation vapour content of the salt or salts we wet the wall somewhat again and measure the surface moisture. This is repeated three times and if we obtain more or less the same measured value for the surface moisture on all three measuring occasions, this measured value can in all probability be assumed to be a very good approximation of the saturation vapour content of the salt or mixture of salts.

## Claims

1. A process for protecting the surface of a material member, preferably a structural member, from the damaging action of water-soluble salts present therein, **characterised** by the following steps
assessing the saturation vapour content of the salt or salts present in the surface layer of the material,
providing a buffer zone between the surface layer and the environment, the climate in the buffer zone being insulated from the environment, and
on the basis of the assessed saturation vapour content, regulating the climate in the buffer zone so that the moisture content in the surface layer reaches a value where the salt or salts assume a state such that their damaging action is essentially eliminated.

2. A process according to claim 1, **characterised** in that the moisture content of the surface layer is assessed by the supplying of moisture to at least part of the same until it is in a saturated state and subsequently the saturation vapour content is measured directly on the material member.

3. A process according to claim 1, **characterised** in that the moisture content of the surface layer is assessed by at least part of it being dried until it is essentially dry and subsequently the saturation vapour content is measured directly on the material member.

4. A process according to any one of the preceding claims, **characterised** in that a buffer zone is provided by the arrangement of a diffusion-inhibiting or diffusion-proof tight barrier between the buffer zone and the environment, which barrier is made of e.g. plastic, metal, or a mineral material.

5. A process according to any one of the preceding claims, **characterised** in that a heat-insulating spacing material is arranged inside or outside the buffer zone, which material preferably has open porosity and is hydrophobic.

6. A process according to claim 5, **characterised** in that glass, mineral wool, plastic, or metal is used as the spacing material.

7. A process according to any one of the preceding claims, **characterised** in that the climate in the buffer zone is regulated by the adjustment of the moisture content therein.

8. A process according to claim 7, **characterised** in that the moisture content of the surface layer of the material is adjusted to a value which essentially corresponds to the saturation vapour content of the salt or salts present in the surface layer.

9. A process according to any one of claims 1-6, **characterised** in that the climate in the buffer zone is regulated by the adjustment of the temperature therein.

10. A process according to any one of claims 1-6, **characterised** in that the climate in the buffer zone is regulated by the adjustment of the vapour pressure therein.

11. A process according to any one of the preceding claims, **characterised** in that the climate in the buffer zone is regulated by the supplying of conditioned air.

12. A process for cleaning a material surface, e.g. the surface of a brick wall which has not been plastered, of water-soluble salts present therein, **characterised** by the following steps
assessing the saturation vapour content of the salt or salts present in the surface layer of the material,
providing a buffer zone between the surface layer and the environment, the climate in the buffer zone being insulated from the environment, and
on the basis of the assessed saturation vapour content, regulating the climate in the buffer zone so that the moisture content in the surface layer reaches a value where the salt or salts assume a state such that they are extracted by migrating through the material towards the surface of the same.

13. A process according to claim 12, **characterised** in that said saturation vapour content of the surface layer is assessed by the addition of moisture to at least part of the same until it is in a saturated state and subsequently the saturation vapour content is measured directly on the material member.

14. A process according to claim 12, **characterised** in that said saturation vapour content of the surface layer is assessed by at least part of it being dried until it is essentially dry and subsequently the saturation vapour content is measured directly on the material member.

15. A process according to any one of the preceding claims, **characterised** in that a buffer zone is provided by the arrangement of a diffusion-inhibiting or diffusion-proof barrier between the buffer zone and the environment, which barrier is made of e.g. plastic, glass, metal or a mineral material.

16. A process according to any one of the preceding claims, **characterised** in that a heat-insulating spacing material is arranged inside or outside the buffer zone, which material preferably has open porosity and is hydrophobic.

17. A process according to claim 16, **characterised** in that glass, mineral wool, plastic or metal is used as the spacing material.

18. A process according to any one of the preceding claims, **characterised** in that the climate in the buffer zone is regulated by the adjustment of the moisture content therein.

19. A process according to claim 18, **characterised** in that the moisture content is adjusted to a value which is about 10% lower, preferably about 5% lower, than saturation vapour content of the salt or salts present in the surface layer.

20. A process according to any one of claims 12-15, **characterised** in that said moisture content in the surface layer is maintained until salts have crystallised and the crystals are collected from the surface.

21. A method for, in the surface layer of a material, preferably the surface layer of a structural member, determining the saturation vapour content of water-soluble salts present therein, **characterised** by the following steps
1) examining said surface layer in order to determine whether it exhibits surface moisture,
2a) if the surface layer is clearly wet, e.g. exhibits surplus moisture, drying a part of this surface,
2b) if the surface layer is clearly dry, wetting a part of this surface,
3) measuring the vapour content of this dried or wetted part of the surface by means of a humidity meter in order to obtain a measured value,
4) optionally repeating steps 2)-3) one or more times,
5) using the measured value obtained in step 3 as an approximation of the saturation vapour content of the salts in the surface layer.

22. A method according to claim 21, **characterised** in that steps 1)-3) are performed iteratively, the drying and the wetting of the surface taking place in small steps, until, under 3), a stable measured value is obtained for the vapour content of the surface, after which this stable measured value is used as a measured value under 5).

23. A method according to claim 21 or 22, **characterised** in that
if the saturation vapour content of surface layer according to 5) deviates from 100% this is considered as a determination that the material contains a water-soluble salt or a mixture of water-soluble salts,
the saturation vapour content according to 5) is used as an approximation of the saturation vapour content of the salt or mixture of salts.

24. A method according to any one of claims 21-23, **characterised** in that
the part of the surface layer which is wetted or dried has an area of between 1 and 5 cm².

25. A process according to any one of claims 1-20, **characterised** in that the saturation vapour content is determined by means of a method according to any one of claims 21-24.

## Patentansprüche

1. Verfahren zum Schützen der Oberfläche eines Materialelements, bevorzugt eines Bauelements, vor der schädigenden Wirkung darin vorhandener wasserlöslicher Salze, gekennzeichnet durch die folgenden Schritte:
Bewerten des Sättigungsdampfgehalts des in der Oberflächenschicht des Materials vorhandenen Salzes bzw. der Salze,
Erzeugen einer Pufferzone zwischen der Oberflächenschicht und der Umgebung, wobei das Klima in der Pufferzone von der Umgebung isoliert wird, und
Regeln des Klimas in der Pufferzone auf der Basis des bewerteten Sättigungsdampfgehalts in der Weise, daß der Feuchtigkeitsgehalt in der Oberflächenschicht einen Wert annimmt, bei welchem das Salz oder die Salze einen Zustand in der Weise annehmen, daß ihre schädigende Wirkung im wesentlichen eliminiert wird.

2. Verfahren nach Anspruch 1, dadurch gekennzeichnet, daß der Feuchtigkeitsgehalt in der Oberfläche bewertet wird, indem Feuchtigkeit zumindest einem Teil derselben zugeführt wird, bis diese sich in einem gesättigten Zustand befindet, und anschließend der Sättigungsdampfgehalt direkt an dem Materialelement gemessen wird.

3. Verfahren nach Anspruch 1, dadurch gekennzeichnet, daß der Feuchtigkeitsgehalt in der Oberfläche bewertet wird, indem zumindest ein Teil davon getrocknet wird, bis sie im wesentlichen trocken ist, und anschließend der Sättigungsdampfgehalt direkt an dem Materialelement gemessen wird.

4. Verfahren nach einem der vorstehenden Ansprüche, dadurch gekennzeichnet, daß eine Pufferzone durch die Anordnung einer eine Diffusion verhindernden oder diffusionsbeständigen dichten Barriere zwischen der Pufferzone und der Umgebung erzeugt wird, wobei die Barriere z.B. aus einem Kunststoff-, Metall-, oder Mineralmaterial besteht.

5. Verfahren nach einem der vorstehenden Ansprüche, dadurch gekennzeichnet, daß ein wärmeisolierendes Abstandsmaterial innerhalb oder außerhalb der Pufferzone angeordnet wird, wobei das Material bevorzugt eine offene Porosität aufweist, und hydrophob ist.

6. Verfahren nach Anspruch 5, dadurch gekennzeichnet, daß Glas, Mineralwolle, Kunststoff oder Metall als das Abstandsmaterial verwendet wird.

7. Verfahren nach einem der vorstehenden Ansprüche, dadurch gekennzeichnet, daß das Klima in der Pufferzone durch die Einstellung des Feuchtigkeitsgehalts darin geregelt wird.

8. Verfahren nach Anspruch 7, dadurch gekennzeichnet, daß der Feuchtigkeitsgehalt der Oberflächenschicht des Materials auf einen Wert eingestellt wird, welcher im wesentlichen dem Sättigungsdampfgehalt des in der Oberfläche vorhandenen Salzes oder der Salze entspricht.

9. Verfahren nach einem der Ansprüche 1 bis 6, dadurch gekennzeichnet, daß das Klima in der Pufferzone durch die Einstellung der Temperatur darin geregelt wird.

10. Verfahren nach einem der Ansprüche 1 bis 6, dadurch gekennzeichnet, daß das Klima in der Pufferzone durch die Einstellung des Dampfdrucks darin geregelt wird.

11. Verfahren nach einem der vorstehenden Ansprüche dadurch gekennzeichnet, daß das Klima in der Pufferzone durch die Zuführung von konditionierter Luft geregelt wird.

12. Verfahren zum Reinigen einer Materialoberfläche, wie z.B. der Oberfläche einer Ziegelwand, welche nicht verputzt ist, von darin vorhandenen wasserlöslichen Salzen, gekennzeichnet durch die folgenden Schritte:
Bewerten des Sättigungsdampfgehalts des in der Oberflächenschicht des Materials vorhandenen Salzes bzw. der Salze,
Erzeugen einer Pufferzone zwischen der Oberflächenschicht und der Umgebung, wobei das Klima in der Pufferzone von der Umgebung isoliert wird, und
Regeln des Klimas in der Pufferzone auf der Basis des bewerteten Sättigungsdampfgehalts in der Weise, daß der Feuchtigkeitsgehalt in der Oberflächenschicht einen Wert annimmt, bei welchem das Salz oder die Salze einen Zustand in der Weise annehmen, daß sie durch ein Durchwandern durch das Material zu dessen Oberfläche extrahiert werden.

13. Verfahren nach Anspruch 12, dadurch gekennzeichnet, daß der Sättigungsdampfgehalt der Oberflächenschicht bewertet wird, indem Feuchtigkeit zumindest einem Teil derselben zugeführt wird, bis diese sich in einem gesättigten Zustand befindet, und anschließend der Sättigungsdampfgehalt direkt an dem Materialelement gemessen wird.

14. Verfahren nach Anspruch 12, dadurch gekennzeichnet, daß der Sättigungsdampfgehalt in der Oberfläche bewertet wird, indem zumindest ein Teil davon getrocknet wird, bis sie im wesentlichen trocken ist, und anschließend der Sättigungsdampfgehalt direkt an dem Materialelement gemessen wird.

15. Verfahren nach einem der vorstehenden Ansprüche, dadurch gekennzeichnet, daß eine Pufferzone durch die Anordnung einer eine Diffusion verhindernden oder diffusionsbeständigen dichten Barriere zwischen der Pufferzone und der Umgebung erzeugt wird, wobei die Barriere aus einem Kunststoff-, Glas-, Metall-, oder Mineralmaterial besteht.

16. Verfahren nach einem der vorstehenden Ansprüche, dadurch gekennzeichnet, daß ein wärmeisolierendes Abstandsmaterial innerhalb oder außerhalb der Pufferzone angeordnet wird, wobei das Material bevorzugt eine offene Porosität aufweist und hydrophob ist.

17. Verfahren nach Anspruch 16, dadurch gekennzeichnet, daß Glas, Mineralwolle, Kunststoff oder Metall als das Abstandsmaterial verwendet wird.

18. Verfahren nach einem der vorstehenden Ansprüche, dadurch gekennzeichnet, daß das Klima in der Pufferzone durch die Einstellung des Feuchtigkeitsgehalts darin geregelt wird.

19. Verfahren nach Anspruch 18, dadurch gekennzeichnet, daß der Feuchtigkeitsgehalt auf einen Wert eingestellt wird, welcher etwa 10% niedriger, bevorzugt etwa 5% niedriger als der Sättigungsdampfgehalt des in der Oberflächenschicht vorhandenen Salzes oder der Salze ist.

20. Verfahren nach einem der vorstehenden Ansprüche 12 bis 15, dadurch gekennzeichnet, daß der Feuchtigkeitsgehalt in der Oberflächenschicht aufrechterhalten wird, bis die Salze kristallisiert sind, und die Kristalle von der Oberfläche gesammelt sind.

21. Verfahren zum Bestimmen, in der Oberflächenschicht eines Materials, bevorzugt in der Oberflächenschicht eines Bauelements, des Sättigungsdampfgehalts von darin vorhandenen wasserlöslichen Salzen, gekennzeichnet durch die folgenden Schritte:
1) Prüfen der Oberflächenschicht, um zu ermitteln, ob sie Oberflächenfeuchtigkeit zeigt,
2a) wenn die Oberflächenschicht deutlich feucht ist, z.B. Überschußfeuchtigkeit zeigt, Trocknen eines Teils dieser Oberfläche,
2b) wenn die Oberflächenschicht deutlich trocken ist, Benetzen eines Teils dieser Oberfläche,
3) Messen des Dampfgehalts dieses getrockneten oder benetzten Teils der Oberfläche mittels eines Feuchtigkeitsmeßgeräts, um einen Meßwert zu erhalten,
4) optionales Wiederholen der Schritte 2)-3) ein oder mehrere Male,
5) Verwenden des in Schritt 3) erhaltenen Meßwerts als eine Annäherung des Sättigungsdampfgehalts des Salzes in der Oberflächenschicht.

22. Verfahren nach Anspruch 21, dadurch gekennzeichnet, daß:
die Schritte 1)-3) wiederholt durchgeführt werden, das Trocknen und Benetzen der Oberfläche in kleinen Schritten stattfindet bis unter 3) ein stabiler Meßwert für den Dampfgehalt der Oberfläche erzielt wird, worauf dann dieser stabile Meßwert als ein Meßwert unter 5) verwendet wird.

23. Verfahren nach Anspruch 21 oder 22, dadurch gekennzeichnet, daß
wenn der Sättigungsdampfgehalt der Oberflächen nach 5) von 100% abweicht, dieses als eine Bestimmung dafür betrachtet wird, daß das Material ein wasserlösliches Salz oder ein Gemisch von wasserlöslichen Salzen enthält,
der Sättigungsdampfgehalt gemäß 5) als eine Annäherung des Sättigungsdampfgehalts des Salzes oder des Gemisches der Salze verwendet wird.

24. Verfahren nach einem der Ansprüche 21 bis 23, dadurch gekennzeichnet, daß
der Teil der Oberflächenschicht, welcher benetzt oder getrocknet wird, eine Fläche zwischen 1 und 5 cm² aufweist.

25. Verfahren nach einem der vorstehenden Ansprüche 1 bis 20, dadurch gekennzeichnet, daß der Sättigungsdampfgehalt mittels eines Verfahrens nach einem der Ansprüche 21 bis 24 ermittelt wird.

## Revendications

1. Processus de protection de la surface d'un élément de matériau, de préférence un élément structurel, vis-à-vis de l'action détériorante de sels solubles dans l'eau présents à l'intérieur, caractérisé par les étapes suivantes consistant à :
évaluer la teneur en vapeur de saturation du sel ou des sels présents dans la couche de surface du matériau,
agencer une zone tampon entre la couche de surface et l'environnement, le climat dans la zone tampon étant isolé de l'environnement, et
sur la base de la teneur en vapeur de saturation évaluée, réguler le climat dans la zone tampon de sorte que la teneur en humidité dans la couche de surface atteint une valeur où le sel ou les sels adoptent un état tel que leur action détériorante est essentiellement éliminée.

2. Processus selon la revendication 1, caractérisé en ce que la teneur en humidité de la couche de surface est évaluée en alimentant de l'humidité dans au moins une partie de celle-ci jusqu'à ce qu'elle soit dans un état saturé et, par la suite, la teneur en vapeur de saturation est mesurée directement sur l'élément de matériau.

3. Processus selon la revendication 1, caractérisé en ce que la teneur en humidité de la couche de surface est évaluée en séchant au moins une partie de celle-ci jusqu'à ce qu'elle soit essentiellement sèche et, par la suite, la teneur en vapeur de saturation est mesurée directement sur l'élément de matériau.

4. Processus selon l'une quelconque des revendications précédentes, caractérisé en ce qu'une zone tampon est agencée du fait de l'agencement d'une barrière imperméable d'inhibition de diffusion ou étanche à la diffusion entre la zone tampon et l'environnement, laquelle barrière est constituée, par exemple, d'une matière plastique, d'un métal, ou d'un matériau minéral.

5. Processus selon l'une quelconque des revendications précédentes, caractérisé en ce qu'un matériau d'espacement thermiquement isolant est agencé à l'intérieur ou à l'extérieur de la zone tampon, lequel matériau a de préférence une porosité ouverte et est hydrophobe.

6. Processus selon la revendication 5, caractérisé en ce que du verre, de la laine de roche, une matière plastique ou un métal est utilisé en tant que matériau d'espacement.

7. Processus selon l'une quelconque des revendications précédentes, caractérisé en ce que le climat dans la zone tampon est régulé par l'ajustement de la teneur en humidité à l'intérieur.

8. Processus selon la revendication 7, caractérisé en ce que la teneur en humidité de la couche de surface du matériau est ajustée à une valeur qui correspond essentiellement à la teneur en vapeur de saturation du sel ou des sels présents dans la couche de surface.

9. Processus selon l'une quelconque des revendications 1 à 6, caractérisé en ce que le climat dans la zone tampon est régulé par l'ajustement de la température à l'intérieur.

10. Processus selon l'une quelconque des revendications 1 à 6, caractérisé en ce que le climat dans la zone tampon est régulé par l'ajustement de la pression de vapeur à l'intérieur.

11. Processus selon l'une quelconque des revendications précédentes, caractérisé en ce que le climat dans la zone tampon est régulé par l'alimentation d'air climatisé.

12. Processus pour nettoyer une surface de matériau, par exemple la surface d'un mur de briques qui n'a pas été enduit de sels solubles dans l'eau présents à l'intérieur, caractérisé par les étapes suivantes consistant à :
évaluer la teneur en vapeur de saturation du sel ou des sels présents dans la couche de surface du matériau,
agencer une zone tampon entre la couche de surface et l'environnement, le climat dans la zone tampon étant isolé de l'environnement, et
sur la base de la teneur en vapeur de saturation évaluée, réguler le climat dans la zone tampon de sorte que la teneur en humidité dans la couche de surface atteint une valeur où le sel ou les sels adoptent un état tel qu'ils sont extraits par migration à travers le matériau en direction de la surface de celui-ci.

13. Processus selon la revendication 12, caractérisé en ce que ladite teneur en vapeur de saturation de la couche de surface est évaluée par l'ajout d'humidité dans au moins une partie de celle-ci jusqu'à ce qu'elle soit dans un état saturé et, par la suite, la teneur en vapeur de saturation est mesurée directement sur l'élément de matériau.

14. Processus selon la revendication 12, caractérisé en ce que ladite teneur en vapeur de saturation de la couche de surface est évaluée par séchage d'au moins une partie de celle-ci jusqu'à ce qu'elle soit essentiellement sèche et, par la suite, la teneur en vapeur de saturation est mesurée directement sur l'élément de matériau.

15. Processus selon l'une quelconque des revendications précédentes, caractérisé en ce qu'une zone tampon est agencée par l'agencement d'une barrière d'inhibition de diffusion ou étanche à la diffusion entre la zone tampon et l'environnement, laquelle barrière est constituée par exemple d'une matière plastique, de verre, d'un métal ou d'un matériau minéral.

16. Processus selon l'une quelconque des revendications précédentes, caractérisé en ce qu'un matériau d'espacement thermiquement isolant est agencé à l'intérieur ou à l'extérieur de la zone tampon, lequel matériau a de préférence une porosité ouverte et est hydrophobe.

17. Processus selon la revendication 16, caractérisé en ce que du verre, de la laine de roche, une matière plastique ou un métal est utilisé en tant que matériau d'espacement.

18. Processus selon l'une quelconque des revendications précédentes, caractérisé en ce que le climat dans la zone tampon est régulé par l'ajustement de la teneur en humidité à l'intérieur.

19. Processus selon la revendication 18, caractérisé en ce que la teneur en humidité est ajustée à une valeur qui est environ 10 % inférieure, de préférence environ 5 % inférieure, à la teneur en vapeur de saturation du sel ou des sels présents dans la couche de surface.

20. Processus selon l'une quelconque des revendications 12 à 15, caractérisé en ce que ladite teneur en humidité dans la couche de surface est maintenue jusqu'à ce que des sels aient cristallisé et les cristaux soient recueillis depuis la surface.

21. Procédé pour déterminer, dans la couche de surface d'un matériau, de préférence la couche de surface d'un élément structurel, la teneur en vapeur de saturation de sels solubles dans l'eau présents à l'intérieur, caractérisé par les étapes suivantes consistant à :
1) examiner ladite couche de surface afin de déterminer si elle présente une humidité en surface,
2a) si la couche de surface est clairement humide, par exemple en présentant une humidité en excès, sécher une partie de cette surface,
2b) si la couche de surface est clairement sèche, humidifier une partie de cette surface,
3) mesurer la teneur en vapeur de cette partie séchée ou humidifiée de la surface par l'intermédiaire d'un dispositif de mesure d'humidité afin d'obtenir une valeur mesurée,
4) répéter de manière facultative les étapes 2)-3) une ou plusieurs fois,
5) utiliser la valeur mesurée obtenue à l'étape 3 en tant qu'approximation de la teneur en vapeur de saturation des sels de la couche de surface.

22. Procédé selon la revendication 21, caractérisé en ce que
les étapes 1)-3) sont effectuées de manière itérative, le séchage et l'humidification de la surface ayant lieu par petites étapes, jusqu'à ce que, à l'étape 3), une valeur mesurée stable soit obtenue pour la teneur en vapeur de la surface, après quoi cette valeur mesurée stable est utilisée en tant que valeur mesurée à l'étape 5).

23. Procédé selon la revendication 21 ou 22, caractérisé en ce que
si la teneur en vapeur de saturation de la couche de surface selon l'étape 5) s'écarte de 100 %, ceci est considéré être une détermination que le matériau contient un sel soluble dans l'eau ou un mélange de sels solubles dans l'eau,
la teneur en vapeur de saturation selon l'étape 5) est utilisée en tant qu'approximation de la teneur en vapeur de saturation du sel ou du mélange de sels.

24. Procédé selon l'une quelconque des revendications 21 à 23, caractérisé en ce que la partie de la couche de surface qui est humidifiée ou séchée a une surface comprise entre 1 et 5 cm².

25. Processus selon l'une quelconque des revendications 1 à 20, caractérisé en ce que la teneur en vapeur de saturation est déterminée par l'intermédiaire d'un procédé selon l'une quelconque des revendications 21 à 24.
